# EUROPÄISCHE PATENTSCHRIFT

(11) **EP 2 534 130 B1**
(45) Veröffentlichungstag und Bekanntmachung des Hinweises auf die Patenterteilung: **07.01.2015**
(21) Anmeldenummer: 11701694.9
(22) Anmeldetag: 04.02.2011
(51) Int. Cl.: C07C 403/24, C07C 45/72

(54) **VERFAHREN ZUR HERSTELLUNG VON ASTAXANTHIN-DIMETHYLDISUCCINAT**
METHOD FOR PRODUCING ASTAXANTHIN DIMETHYL DISUCCINATE
PROCÉDÉ DE PRODUCTION D'ASTAXANTHINE DIMÉTHYLE DISUCCINATE

(30) Priorität: 08.02.2010 EP 10152908
(43) Veröffentlichungstag der Anmeldung: 19.12.2012
(73) Patentinhaber: BASF SE, 67056 Ludwigshafen (DE)
(72) Erfinder: ERNST, Hansgeorg, 67346 Speyer (DE); DOBLER, Walter, 68723 Schwetzingen (DE); KELLER, Andreas, 67346 Speyer (DE); HENRICH, Klaus, 67454 Haßloch (DE)
(86) Internationale Anmeldenummer: PCT/EP2011/051618
(87) Internationale Veröffentlichungsnummer: WO 2011/095571

(56) Entgegenhaltungen:
- WO-A1-03/066583
- WO-A1-2007/128574

## Beschreibung

Die vorliegende Erfindung betrifft ein verbessertes Verfahren zur Herstellung von all-E Astaxanthin-dimethyldisuccinat der Formel I.

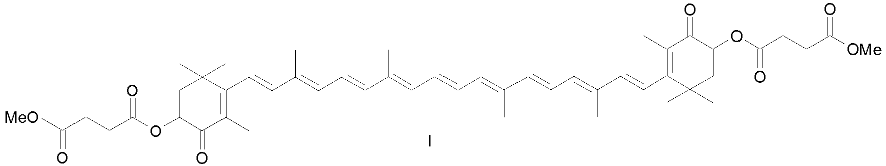

Astaxanthin selbst ist ein begehrter Farbstoff, der vor allem als Futtermittelzusatzstoff für die Pigmentierung von Zuchtlachs verwendet wird.

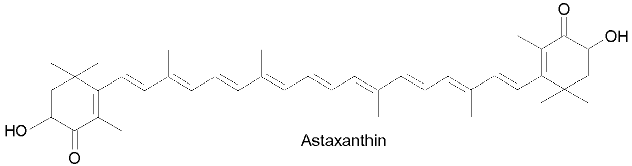

Eine sehr effiziente Methode zur Herstellung von Astaxanthin ist die doppelte Wittig-Olefinierung des symmetrischen C₁₀-Dialdehyds der Formel II mit zwei Äquivalenten des entsprechenden C₁₅-Phosphoniumsalzes der Formel III so wie es unter anderem in Helv. Chim. Acta 64, 7, 2445 (1981) beschrieben wird.

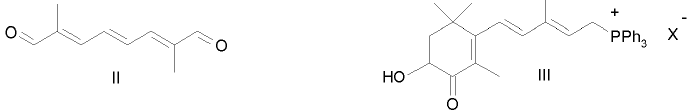

Diese Umsetzung kann beispielsweise in Dichlormethan unter Verwendung von methanolischer Natriummethylat-Lösung als Base (vgl. auch WO 2007128574) oder durch Erhitzen der Komponenten der Formeln II und III in 1,2-Epoxybutan durchgeführt werden.

Zur Verbesserung der Lagerstabilität von Astaxanthin im Fischfutter werden in WO 03/066583 verschiedene Diester des Astaxanthins vorgeschlagen, wobei unter anderem Astaxanthin-dimethyldisuccinat der Formel I genannt wird.

Zur Herstellung der Diester des Astaxanthins wird gemäß WO 03/066583 kristallines Astaxanthin in einem inerten Lösungsmittel mit einem Carbonsäurechlorid oder einem Carbonsäureanhydrid in Gegenwart einer organischen Base umgesetzt (WO 03/066583, Seite 7, Zeilen 3 bis 20). Bei der Veresterung des Astaxanthins mit einer freien Säure erfolgt die Umsetzung in Gegenwart eines Dehydratisierungsreagenzes (WO 03/066583, Seite 7, Zeilen 21 bis 28). Astaxanthin-dimethyldisuccinat der Formel I wurde gemäß dem Beispiel 5 in der WO 03/066583 (Seite 13, Zeile 13 bis 25) in einer Ausbeute von 49,9% und einer Reinheit von 79,3% gewonnen. Durch anschließende Rekristallisation aus Methylenchlorid / Methanol konnte der Diester in einer Reinheit von 98% (nach HPLC) erhalten werden.

Nachteilig an diesem Vorgehen ist, dass als Ausgangsmaterial für die Veresterung kristallines Astaxanthin eingesetzt wird. Die Herstellung von kristallinem Astaxanthin als Zwischenprodukt für die Veresterung bedeutet einen erheblichen Produktionsaufwand für Kristallisation, Filtration, Waschung, Trocknung, Abfüllung, Lagerung und Dosierung des Feststoffs in der Folgestufe. Zudem entstehen bei der Kristallisation von Astaxanthin Ausbeuteverluste durch die Restlösllichkeit des Wertproduktes in der Mutterlauge.

Es war Aufgabe der vorliegenden Erfindung, die Nachteile der bekannten Synthese von Astaxanthin-dimethyldisuccinat der Formel I zu verbessern. Einerseits sollte das Verfahren zur Herstellung von Astaxanthin-dimethyldisuccinat der Formel I vereinfacht werden und zum anderen die Ausbeute und Reinheit an Astaxanthindimethyldisuccinat der Formel I verbessert werden.

Diese Aufgabe wird durch ein Verfahren zur Herstellung von all-E Astaxanthindimethyldisuccinat der Formel I 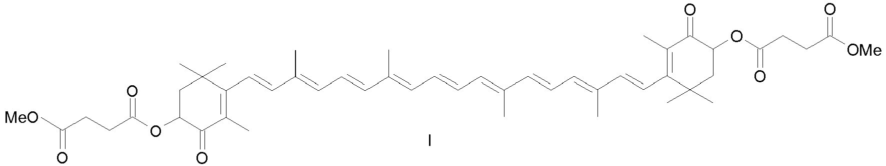 umfassend die Verfahrensschritte:
a) Umsetzung eines C₁₀-Dialdehyds der Formel II 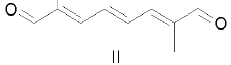 in einer doppelten Wittig Reaktion mit einem C₁₅-Phosphoniumsalz der Formel III 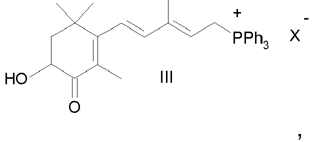 worin X⁻ das Anion einer organischen oder anorganischen Säure HX ist,
   in einem Lösungsmittel umfassend Dichlormethan, durch Zugabe einer Lösung eines Alkali- oder Erdalkalimetallalkoholates M¹OR¹ oder M²(OR¹)₂ in einem Alkohol R¹OH als Base,
   worin
   M¹ gleich Li, Na, K oder Rb,
   M² gleich Mg, Ca, Sr oder Ba
   und
   R¹ gleich Methyl, Ethyl, n-Propyl oder iso-Propyl ist,
   zu einem Astaxanthin der Formel IV 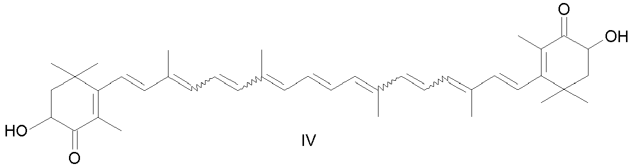 wobei die beiden neu gebildeten Doppelbindungen sowohl in der Z-Konfiguration als auch in der E-Konfiguration vorliegen,
   gegebenenfalls gefolgt von einer wässrigen Aufarbeitung des Reaktionsgemisches zur Entfernung der Salze M¹X oder M²X₂;
b) Entfernen des Alkohols R¹OH aus dem Reaktionsgemisch des Verfahrensschrittes a) durch wiederholtes Waschen des Reaktionsgemisches mit Wasser oder durch Abdestillieren eines Lösungsmittelgemisches enthaltend Dichlormethan und den Alkohol R¹OH,
   gegebenenfalls gefolgt vom Trocknen des Reaktionsgemisches durch Entfernen von Wasser mittels azeotroper Destillation von Dichlormethan und Wasser;
c) Umsetzung des Astaxanthins der Formel IV, welches in dem in Verfahrensschritt b) behandelten Reaktionsgemisch vorliegt, in Dichlormethan durch Zugabe von Methylsuccinoylchlorid der Formel V 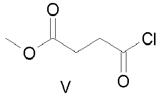 und einer organischen, stickstoffhaltigen Base zu einem Astaxanthindimethyldisuccinat der Formel la ; 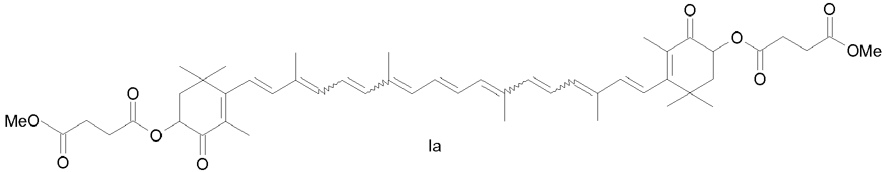
d) Wässrige Aufarbeitung des Reaktionsgemisches aus Verfahrensschritt c) zur Entfernung von wasserlöslichen Salzen;
e) Lösungsmittelaustausch von Dichlormethan durch einen Alkohol R²OH durch Entfernen des Dichlormethans aus dem Reaktionsgemisch des Verfahrensschrittes d) mittels Destillation und Zugabe des Alkohols R²OH zu dem Reaktionsgemisch, wobei R² gleich Methyl, Ethyl, n-Propyl oder i-Propyl ist;
f) thermische Isomerisierung des in Verfahrensschritt c) hergestellten Astaxanthindimethyldisuccinats der Formel la zum all-E Astaxanthin-dimethyldisuccinat der Formel I durch Erhitzen des R²OH haltigen Reaktionsgemisches aus Verfahrensschritt e) auf mindestens 50°C, wobei das Reaktionsgemisch einen Wassergehalt von 10 bis 90 Vol.-% Wasser bezogen auf das Gesamtvolumen des Reaktionsgemisches aufweist, und wobei das all-E Astaxanthin-dimethyldisuccinat der Formel I in kristalliner Form anfällt und anschließend gegebenenfalls isoliert wird;
gelöst.

In Verfahrensschritt a) des erfindungsgemäßen Verfahrens wird ein C₁₀-Dialdehyd der Formel II 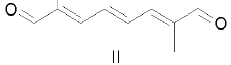 in einer doppelten Wittig Reaktion mit einem C₁₅-Phosphoniumsalz der Formel III 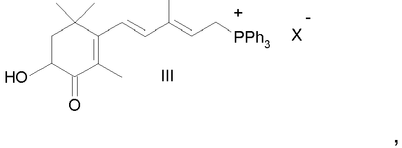 worin X⁻ das Anion einer organischen oder anorganischen Säure HX ist, wie beispielsweise Chlorwasserstoffsäure, Bromwasserstoffsäure oder lodwasserstoffsäure, eine Sulfonsäure, wie Methansulfonsäure, p-Toluolsulfonsäure oder Trifluormethansulfonsäure, oder eine Trihalogenessigsäure, wie Trifluoressigsäure oder Trichloressigsäure,

in einem Lösungsmittel umfassend Dichlormethan, durch Zugabe einer Lösung eines Alkali- oder Erdalkalimetallalkoholates M¹OR¹ oder M²(OR¹)₂, bevorzugt eines Alkalimetallalkoholates M¹OR¹ in einem Alkohol R¹OH als Base,
worin
- M¹: gleich Li, Na, K oder Rb, bevorzugt Li oder Na, insbesondere Na,
- M²: gleich Mg, Ca, Sr oder Ba, bevorzugt Mg
und
- R¹: gleich Methyl, Ethyl, n-Propyl oder iso-Propyl, bevorzugt Methyl oder Ethyl, insbesondere Methyl ist,
zu einem Astaxanthin der Formel IV 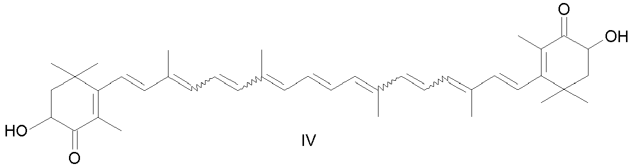 wobei die beiden neu gebildeten Doppelbindungen sowohl in der Z-Konfiguration als auch in der E-Konfiguration vorliegen, umgesetzt, gegebenenfalls gefolgt von einer anschließenden wässrigen Aufarbeitung des Reaktionsgemisches zur Entfernung der Salze M¹X oder M²X₂.

Bevorzugt handelt es sich bei dem Anion X- um Chlorid oder Bromid.
In Verfahrensschritt a) werden bevorzugt zunächst der C₁₀-Dialdehyd der Formel II und das C₁₅-Phosphoniumsalz der Formel III in einem Lösungsmittel enthaltend mehr als 90 Vol.-%, besonders bevorzugt mehr als 95 Vol.-% Dichlormethan bezogen auf das Gesamtvolumen des Lösungsmittels, insbesondere in reinem Dichlormethan, das heißt Dichlormethan mit einer Reinheit von größer 99 Vol.-%, gelöst.

Anschließend werden in Verfahrensschritt a) bevorzugt die Verbindungen der Formeln II und III durch Zugabe einer Lösung von Natriummethanolat in Methanol als Base zum Astaxanthin der Formel IV kondensiert.

In dem Astaxanthin der Formel IV liegen die beiden neu gebildeten Doppelbindungen sowohl in der Z-Konfiguration als auch in der E-Konfiguration vor. Überwiegend, das heißt zu mehr als 50%, besitzen die beiden neu gebildeten Doppelbindungen in dem Astaxanthin der Formel IV die E, E Konfiguration. Der Anteil der neu gebildeten Doppelbindungen mit Z-Konfiguration beträgt in dem Gemisch des Astaxanthins der Formel IV bis zu 25 %.

Bevorzugt wird in Verfahrensschritt a) das Reaktionsgemisch zur Entfernung der Salze M¹X oder M²X₂ einer wässrigen Aufarbeitung unterworfen.

Nach der wässrigen Aufarbeitung wird als Reaktionsgemisch eine rohe Lösung von Astaxanthin der Formel IV in Dichlormethan erhalten, welche wassergesättigt ist und Reste an dem Alkohol R¹OH, insbesondere Methanol enthält.

In Verfahrensschritt b) des erfindungsgemäßen Verfahrens wird der Alkohol R¹OH aus dem Reaktionsgemisch des Verfahrensschrittes a) durch wiederholtes Waschen des Reaktionsgemisches mit Wasser oder durch Abdestillieren eines Lösungsmittelgemisches enthaltend Dichlormethan und den Alkohol R¹OH, insbesondere durch Abdestillieren des Lösungsmittelgemisches enthaltend Dichlormethan und den Alkohol R¹OH, entfernt, gegebenenfalls gefolgt vom Trocknen des Reaktionsgemisches durch Entfernen von Wasser mittels azeotroper Destillation von Dichlormethan und Wasser.

Bevorzugt wird in Verfahrensschritt b) durch mehrmaliges Andestillieren, Waschen des Destillates mit Wasser und Rückführen des gewaschenen Destillats in die Astaxanthin-Rohlösung der Alkohol R¹OH, insbesondere Methanol, entfernt.

Im Falle von Methanol kann die Entfernung des Methanols aus dem Reaktionsgemisch wie vorangehend beschrieben diskontinuierlich in mehreren Stufen erfolgen. In einem technischen Verfahren wird die Entfernung des Methanols aus dem Reaktionsgemisch vorzugsweise kontinuierlich durchgeführt, indem das Methanol aus dem Reaktionsgemisch durch kontinuierliche Waschung des Methanol enthaltenden Dichlormethan-Destillats mit Wasser in einer Mixer-/Settler-Apparatur entfernt wird. Dabei wird die organische Unterphase kontinuierlich in die Astaxanthin-Rohlösung rückgeführt, bis die Astaxanthin-Rohlösung methanolfrei ist.

Bevorzugt wird nach erfolgter Methanolabtrennung die wassergesättigte Astaxanthin-Rohlösung nach dem Fachmann bekannten Methoden getrocknet, bevorzugt durch Azeotropdestillation (Dichlormethan/Wasser-Heteroazeotrop). Dies kann durch einfaches Andestillieren oder durch Auskreisen von Wasser mittels eines entsprechend konstruierten Wasserabscheiders erfolgen.

Alle Destillationsschritte werden vorzugsweise beim Normaldruck durchgeführt, können aber auch bei Unterdruck stattfinden. Aus praktischen Gründen (Kondensierbarkeit der Dichlormethan-Aezotrope) wird man jedoch nicht unter 300 mbar arbeiten.

In Verfahrensschritt c) des erfindungsgemäßen Verfahrens wird das Astaxanthin der Formel IV, welches in dem in Verfahrensschritt b) behandelten Reaktionsgemisch vorliegt, in Dichlormethan durch Zugabe von Methylsuccinoylchlorid der Formel V 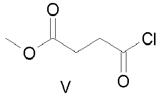 und einer organischen, stickstoffhaltigen Base zu einem Astaxanthin-dimethyldisuccinat der Formel la 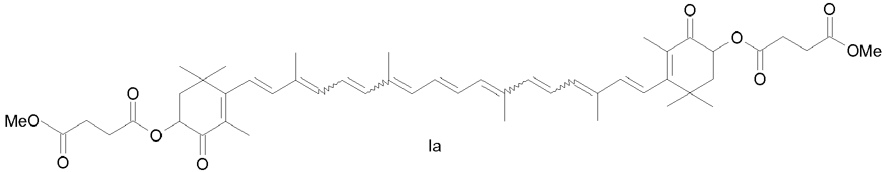 umgesetzt.

Die in Verfahrensschritt b) erhaltene R¹OH-freie, bevorzugt auch wasserfreie, insbesondere methanol- und wasserfreie Dichlormethan-Lösung von Roh-Astaxanthin wird in an sich bekannter Weise mit dem Methylsuccinoychlorid der Formel V in Gegenwart einer organischen Base zu dem Astaxanthin-dimethyldisuccinat der Formel la umgesetzt. Wasserfrei bzw. R¹OH-frei bedeutet in diesem Zusammenhang einen Wassergehalt bzw. einen R¹OH-Gehalt in dem Reaktionsgemisch von weniger als 0,2 Gew.-%, bevorzugt weniger als 0,1 Gew.-%, insbesondere weniger als 0,075 Gew.-% Wasser bzw. R¹OH bezogen auf die Masse des Reaktionsgemisches.

Als organische, stickstoffhaltige Base können beispielsweise Amine, wie primäre, sekundäre oder tertiär Alkyl- oder Arylamine, oder basische stickstoffhaltige Heteroaromaten wie Pyridin oder Pyridinderivate verwendet werden. Bevorzugt werden als Base ein Trialkylamin wie etwa Triethylamin, Pyridin oder ein Dialkylaminopyridin wie etwa 4-Dimethylaminopyridin verwendet. Besonders bevorzugt ist in Verfahrensschritt c) die organische, stickstoffhaltige Base Pyridin oder ein Pyridin-Derivat.

Das molare Verhältnis Astaxanthin : Methylsuccinoylchlorid liegt im Bereich von 1 : 2,0 - 1 : 3,0, vorzugsweise bei 1 : 2,1 - 1 : 2,5. Die Base wird mindestens stöchiometrisch zum Säurechlorid, vorzugsweise aber in einem Überschuss von 10-50 mol-%, bezogen auf Methylsuccinoylchlorid, eingesetzt. Die Reaktionstemperatur kann von - 10°C bis zur Rückflußtemperatur des Reaktionsgemisches liegen. Vorzugsweise wird die Reaktion im Temperaturbereich von 0 bis 25°C durchgeführt.

In Verfahrensschritt d) des erfindungsgemäßen Verfahrens wird das Reaktionsgemisch aus Verfahrensschritt c) zur Entfernung von wasserlöslichen Salzen wässrig aufgearbeitet. Dabei werden die Hydrochloride der organischen, stickstoffhaltigen Base weitestgehend, sowie eventuell noch vorhandene, in Verfahrensschritt a) entstandene Salze M¹X oder M²X₂ aus dem Dichlormethan-haltigen Reaktionsgemisch entfernt.

In Verfahrensschritt e) des erfindungsgemäßen Verfahrens wird das Lösungsmittel Dichlormethan durch einen Alkohol R²OH durch Entfernen des Dichlormethans aus dem Reaktionsgemisch des Verfahrensschrittes d) mittels Destillation und Zugabe des Alkohols R²OH zu dem Reaktionsgemisch, wobei R² gleich Methyl, Ethyl, n-Propyl oder i-Propyl, insbesondere gleich Methyl ist, ausgetauscht.

Bevorzugt ist in Verfahrensschritt e) der Alkohol R²OH gleich Methanol.

Der Lösungsmittelaustausch kann schrittweise vorgenommen werden, indem eine bestimmte Menge Dichlormethan abdestilliert wird und durch eine bestimmte Menge Alkohol R²OH ersetzt wird und dieses Vorgehen so lange wiederholt wird, bis das Dichlormethan in der gewünschten Menge, bevorzugt vollständig, entfernt wurde. Alternativ kann das durch Destillation entfernte Dichlormethan auch kontinuierlich durch das entsprechende Volumen des Alkohols R²OH ersetzt werden (isochore Fahrweise). Vollständige Entfernung von Dichlormethan bedeutet im obigen Zusammenhang, dass der Restgehalt an Dichlormethan kleiner als 0,5 Gew.-%, bevorzugt kleiner als 0,1 Gew.-%, insbesondere weniger als 0,05 Gew.-% Dichlormethan bezogen auf die Masse des Reaktionsgemisches ist.

Der Lösungsmittelaustausch wird vorgenommen, um das Astaxanthin-dimethyldisuccinat der Formel la und insbesondere das all-E Astaxanthin-dimethyldisuccinat der Formel I, welche in Dichlormethan gut löslich sind, aus einem Alkohol R²OH auskristallisieren zu können, wobei das noch vorhandene Triphenylphoshinoxid aus Verfahrensschritt a) in Lösung bleibt und somit vom kristallinen Produkt gut abtrennbar ist.

Bevorzugt wird dem Alkohol R²OH, insbesondere Methanol, Wasser zugegeben, wobei die Wassermenge so gewählt wird, dass das Triphenylphoshinoxid noch nicht ausfällt. Prinzipiell kann das Wasser, zumindest teilweise, schon vor dem Lösungsmittelaustausch (Verfahrensschritt e)) zu dem Reaktionsgemisch gegeben worden sein. Zur einfacheren Berechung und Bestimmung der benötigten Wassermenge für einen gewünschten Wassergehalt, wird das Wasser erst nach dem Lösungsmittelaustausch von Dichlormethan gegen den Alkohol R²OH, insbesondere gegen Methanol, zu dem Reaktionsgemisch gegeben. Bevorzugt wird ein Wassergehalt von 10 bis 90 Vol.-%, besonders bevorzugt 30 bis 70 Vol.-%, ganz besonders bevorzugt 40 bis 60 Vol.-%, insbesondere 45 bis 55 Vol.-% Wasser bezogen auf das Gesamtvolumen des Reaktionsgemisches eingestellt.

In Verfahrensschritt f) des erfindungsgemäßen Verfahrens wird das in Verfahrensschritt c) hergestellte Astaxanthin-dimethyldisuccinat der Formel la durch Erhitzen des R²OH haltigen Reaktionsgemisches aus Verfahrensschritt e) auf mindestens 50°C zum all-E Astaxanthin-dimethyldisuccinat der Formel I thermisch isomerisiert, wobei das Reaktionsgemisch einen Wassergehalt von 10 bis 90 Vol.-%, besonders bevorzugt 30 bis 70 Vol.-%, ganz besonders bevorzugt 40 bis 60 Vol.-%, insbesondere 45 bis 55 Vol.-% Wasser bezogen auf das Gesamtvolumen des Reaktionsgemisches aufweist, und wobei das all-E Astaxanthin-dimethyldisuccinat der Formel I in kristalliner Form anfällt und anschließend gegebenenfalls isoliert wird.

Das Reaktionsgemisch aus Verfahrensschritt e) wird bevorzugt bei einer Temperatur zwischen 80 und 120°C, besonders bevorzugt zwischen 90 und 110°C isomerisiert. Falls die gewünschte Temperatur oberhalb des Siedepunktes des Lösungmittels bzw. des Lösungsmittelsgemisches unter Atmosphärendruck liegt, wird das Reaktionsgemisch in einem geschlossenen System erhitzt, das für den sich aufbauenden Überdruck geeignet ist.

Bevorzugt wird in Verfahrensschritt f) die thermische Isomerisierung in wässrigem Methanol zwischen 80 und 120°C, besonders bevorzugt zwischen 90 und 110°C unter Eigendruck in einer geschlossenen Apparatur durchgeführt wird.

Das Reaktionsgemisch wird üblicherweise für 1 bis 20 h bei einer Temperatur zwischen 80 und 120°C thermisch isomerisiert. Bei niedrigeren Temperaturen kann diese Zeit auch länger sein.

Bevorzugt wird der in Verfahrensschritt f) definierte Wassergehalt durch Zugabe von Wasser zum Reaktionsgemisch im Anschluss an Verfahrensschritt e) eingestellt.

Nach der thermischen Isomerisierung wird das Gemisch üblicherweise auf O bis 25°C, bevorzugt 0 bis 12°C abgekühlt und das auskristallisierte all-E Astaxanthindimethyldisuccinat der Formel I kann in hoher Ausbeute und Reinheit durch Filtration isoliert werden.

Das vorangehend beschriebene erfindungsgemäße Verfahren vermeidet die Nachteile des zweistufigen Prozesses, bei dem zuerst all-E Astaxanthin isoliert wird und dieses kristalline Produkt anschließend zum all-E Astaxanthin-dimethyldisuccinat der Formel I umgesetzt wird. Überraschenderweise zeigte sich, dass ein aus dem C₁₀-Dialdehyd der Formel II und dem C₁₅-Phosphoniumsalz der Formel III hergestelltes Astaxanthin der Formel IV für die Überführung in Astaxanthin-dimethyldisuccinat der Formel la nicht in Substanz isoliert werden muss, sondern als Rohlösung in Gegenwart von Triphenylphosphinoxid und anderen Verunreinigungen aus der Synthese direkt mit Methylsuccinoylchlorid der Formel V (4-Chlor-4-oxo-buttersäuremethylester) zu Astaxanthindimethyldisuccinat der Formel la umgesetzt werden kann, und daraus nach thermischer Isomerisierung all-E Astaxanthin-dimethyldisuccinat der Formel I in hoher Ausbeute und Reinheit in kristalliner Form gewonnen werden kann.

Die Erfindung wird durch folgendes, die Erfindung jedoch nicht einschränkendes Beispiel erläutert.

### Beispiel 1

Die Astaxanthin-Rohlösung wurde durch Umsetzung von II mit III in Dichlormethan unter Verwendung von methanolischer Natriummethylatlösung (vgl. Helv. Chim. Acta 64, 7, 2445 (1981) hergestellt. Die Lösung enthielt 4,56 Gew.-% Astaxanthin (HPLC-Analyse) und 0,8 Gew.-% Methanol (GC-Analyse) sowie 0,55 Gew.-% Wasser (Karl-Fischer-Titration).

982 g dieser Lösung (entsprechend 75 mmol Astaxanthin) wurden vorgelegt. Bei Normaldruck wurden über einen Normag-Kolonnenkopf 350 ml Lösungsmittel abdestilliert. Die wässrige Oberphase des Destillats wurde abgetrennt, und die Unterphase mit 175 g Wasser gewaschen. Die Phasen wurden getrennt, und die organische Unterphase wurde in den Destillationssumpf rückgeführt.

Dieses Prozedere wurde noch zweimal wiederholt. Anschließend wurden 350 ml Lösungsmittel bei Normaldruck abdestilliert. Der verbleibende Destillationsrückstand enthielt kein Methanol mehr. Der Wassergehalt lag bei 0,051 Gew.-%.

Zum Destillationsrückstand wurden bei 20°C 21,36 g (270 mmol) Pyridin gegeben. Anschließend wurden bei 20°C 34,92 g (225,0 mmol) Methylsuccinoylchlorid (4-Chlor-4-oxobuttersäure-methylester) in 30 min. zugetropft. Das Reaktionsgemisch wurde 3h bei 20-25°C nachgerührt. Dann wurde das Reaktionsgemisch durch Zugabe von 100 ml Wasser hydrolysiert. Die wässrige Phase wurde abgetrennt und die organische Phase zweimal mit je 100 ml Wasser gewaschen. Anschließend wurde Dichlormethan über einem Normag-Kolonnenkopf abdestilliert. Das Destillat wurde simultan durch Methanol ersetzt (isochore Fahrweise), bis die Übergangstemperatur 65°C erreicht hatte. Das Gemisch wurde mit 300 ml Wasser versetzt, 4h bei 100°C unter Eigendruck nachgerührt, auf 10°C abgekühlt und 1 h bei 10°C ausgerührt. Das ausgefallene Produkt wurde abfiltriert, zweimal mit je 50 ml kaltem (10°C) Methanol gewaschen und über Nacht im Vakuumtrockenschrank bei 50°C bis 10 mbar getrocknet.

| | |
|---|---|
| Auswaage: | 52,5 g Ax-DMDS (84,9% bez. Astaxanthin in der Rohlösung) |
| Fp: | 115-117°C |
| Reinheit: | 94,6% (HPLC-Flächen-%) |

## Patentansprüche

1. Verfahren zur Herstellung von all-E Astaxanthin-dimethyldisuccinat der Formel I 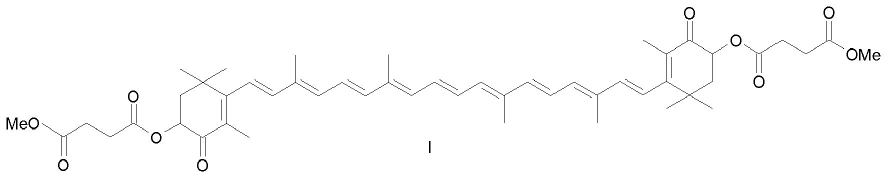 umfassend die Verfahrensschritte:
a) Umsetzung eines C₁₀-Dialdehyds der Formel II 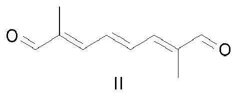 in einer doppelten Wittig Reaktion mit einem C₁₅-Phosphoniumsalz der Formel III 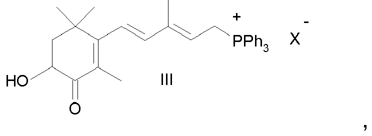 worin X⁻ das Anion einer organischen oder anorganischen Säure HX ist,
in einem Lösungsmittel umfassend Dichlormethan, durch Zugabe einer Lösung eines Alkali- oder Erdalkalimetallalkoholates M¹OR¹ oder M²(OR¹)₂ in einem Alkohol R¹OH als Base,
worin
M¹ gleich Li, Na, K oder Rb,
M² gleich Mg, Ca, Sr oder Ba
und
R¹ gleich Methyl, Ethyl, n-Propyl oder iso-Propyl ist,
zu einem Astaxanthin der Formel IV 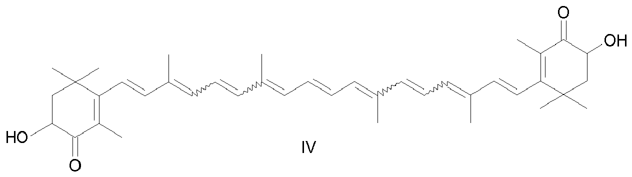 wobei die beiden neu gebildeten Doppelbindungen sowohl in der Z-Konfiguration als auch in der E-Konfiguration vorliegen,
gegebenenfalls gefolgt von einer wässrigen Aufarbeitung des Reaktionsgemisches zur Entfernung der Salze M¹X oder M²X₂;
b) Entfernen des Alkohols R¹OH aus dem Reaktionsgemisch des Verfahrensschrittes a) durch wiederholtes Waschen des Reaktionsgemisches mit Wasser oder durch Abdestillieren eines Lösungsmittelgemisches enthaltend Dichlormethan und den Alkohol R¹OH,
gegebenenfalls gefolgt vom Trocknen des Reaktionsgemisches durch Entfernen von Wasser mittels azeotroper Destillation von Dichlormethan und Wasser;
c) Umsetzung des Astaxanthins der Formel IV, welches in dem in Verfahrensschritt b) behandelten Reaktionsgemisch vorliegt, in Dichlormethan durch Zugabe von Methylsuccinoylchlorid der Formel V 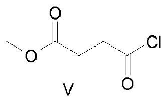 und einer organischen, stickstoffhaltigen Base zu einem Astaxanthindimethyldisuccinat der Formel la ; 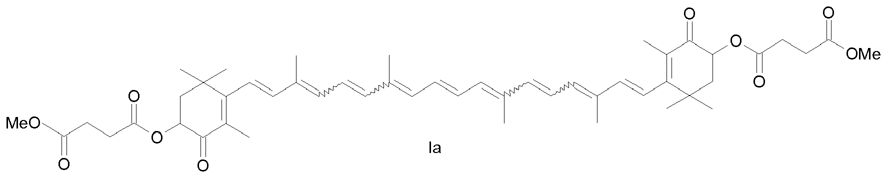
d) Wässrige Aufarbeitung des Reaktionsgemisches aus Verfahrensschritt c) zur Entfernung von wasserlöslichen Salzen;
e) Lösungsmittelaustausch von Dichlormethan durch einen Alkohol R²OH durch Entfernen des Dichlormethans aus dem Reaktionsgemisch des Verfahrensschrittes d) mittels Destillation und Zugabe des Alkohols R²OH zu dem Reaktionsgemisch, wobei R² gleich Methyl, Ethyl, n-Propyl oder i-Propyl ist;
f) thermische Isomerisierung des in Verfahrensschritt c) hergestellten Astaxanthin-dimethyldisuccinats der Formel la zum all-E Astaxanthindimethyldisuccinat der Formel I durch Erhitzen des R²OH haltigen Reaktionsgemisches aus Verfahrensschritt e) auf mindestens 50°C, wobei das Reaktionsgemisch einen Wassergehalt von 10 bis 90 Vol.-% Wasser bezogen auf das Gesamtvolumen des Reaktionsgemisches aufweist, und wobei das all-E Astaxanthin-dimethyldisuccinat der Formel I in kristalliner Form anfällt und anschließend gegebenenfalls isoliert wird.

2. Verfahren nach Anspruch 1, wobei in Verfahrensschritt a) die Base eine Lösung von Natriummethanolat in Methanol ist.

3. Verfahren nach Anspruch 2, wobei in Verfahrensschritt b) das Methanol aus dem Reaktionsgemisch durch kontinuierliche Waschung des Methanol enthaltenden Dichlormethan-Destillats mit Wasser in einer Mixer-/Settler-Apparatur entfernt wird.

4. Verfahren nach einem der Ansprüche 1 bis 3, wobei in Verfahrensschritt c) die organische, stickstoffhaltige Base Pyridin oder ein Pyridin-Derivat ist.

5. Verfahren nach einem der Ansprüche 1 bis 4, wobei in Verfahrensschritt e) der Alkohol R²OH gleich Methanol ist.

6. Verfahren nach Anspruch 5, wobei in Verfahrensschritt f) die thermische Isomerisierung in wässrigem Methanol zwischen 80 und 120°C unter Eigendruck in einer geschlossenen Apparatur durchgeführt wird.

7. Verfahren nach einem der Ansprüche 1 bis 6, wobei der in Verfahrensschritt f) definierte Wassergehalt durch Zugabe von Wasser zum Reaktionsgemisch im Anschluss an Verfahrensschritt e) eingestellt wird.

## Claims

1. A method for producing all-E astaxanthin dimethyldisuccinate of the formula I 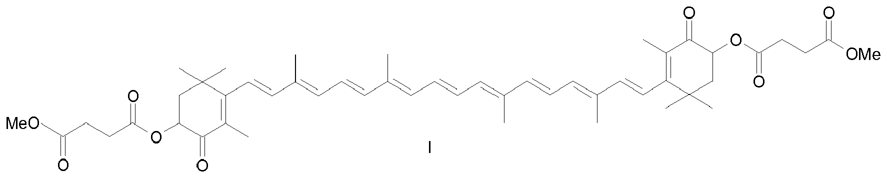 which comprises the process steps:
a) reacting a C₁₀ dialdehyde of the formula II 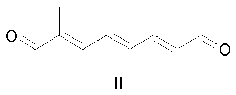 in a double Wittig reaction with a C₁₅ phosphonium salt of the formula III 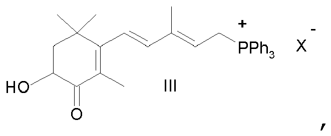 where X⁻ is the anion of an organic or inorganic acid HX,
in a solvent comprising dichloromethane by adding a solution of an alkali metal alkoxide or alkaline earth metal alkoxide M¹OR¹ or M²(OR¹)₂ in an alcohol R¹OH as base,
where
M¹ is Li, Na, K or Rb,
M² is Mg, Ca, Sr or Ba
and
R¹ is methyl, ethyl, n-propyl or isopropyl,
to give an astaxanthin of the formula IV 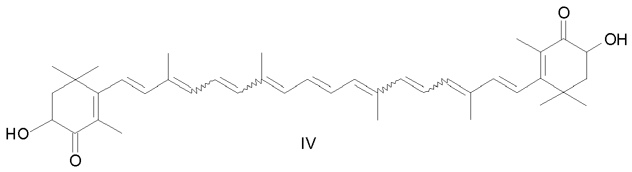 wherein the two new double bonds formed are not only present in the Z configuration but also in the E configuration,
optionally followed by an aqueous workup of the reaction mixture for removing the salts M¹X or M²X²;
b) removing the alcohol R¹OH from the reaction mixture of process step a) by repeated washing of the reaction mixture with water or by distilling off a solvent mixture comprising dichloromethane and the alcohol R¹OH,
optionally followed by drying the reaction mixture by removing water by means of azeotropic distillation of dichloromethane and water;
c) reacting the astaxanthin of the formula IV which is present in the reaction mixture treated in process step b) in dichloromethane by adding methyl succinoyl chloride of the formula V 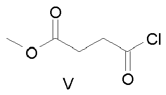 and an organic nitrogenous base to form an astaxanthin dimethyldisuccinate of the formula Ia; 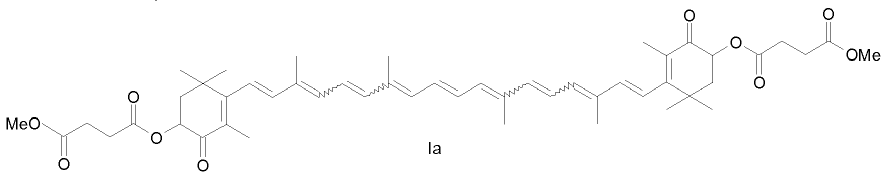
d) an aqueous workup of the reaction mixture of process step c) for removing water-soluble salts;
e) solvent exchange of dichloromethane by an alcohol R²OH by removing the dichloromethane from the reaction mixture of process step d) by distillation and adding the alcohol R²OH to the reaction mixture, wherein R² is methyl, ethyl, n-propyl or isopropyl;
f) thermal isomerization of the astaxanthin dimethyldisuccinate of the formula Ia produced in process step c) to give the all-E astaxanthin dimethyldisuccinate of the formula I by heating the R²OH-comprising reaction mixture of process step e) to at least 50°C, wherein the reaction mixture has a water content of 10 to 90% by volume of water based on the total volume of the reaction mixture, and wherein the all-E astaxanthin dimethyldisuccinate of the formula I occurs in crystalline form and is subsequently optionally isolated.

2. The method according to claim 1, wherein, in process step a) the base is a solution of sodium methoxide in methanol.

3. The method according to claim 2, wherein, in process step b), the methanol is removed from the reaction mixture by continuous washing of the methanol-comprising dichloromethane distillate with water in a mixer/settler apparatus.

4. The method according to any one of claims 1 to 3, wherein, in process step c), the organic nitrogenous base is pyridine or a pyridine derivative.

5. The method according to any one of claims 1 to 4, wherein, in process step e), the alcohol R²OH is methanol.

6. The method according to claim 5, wherein, in process step f), the thermal isomerization is carried out in aqueous methanol between 80 and 120°C under the inherent pressure in a closed apparatus.

7. The method according to any one of claims 1 to 6, wherein the water content defined in process step f) is set by adding water to the reaction mixture subsequently to process step e).

## Revendications

1. Procédé de fabrication de disuccinate diméthylique d'astaxanthine all-E de formule I 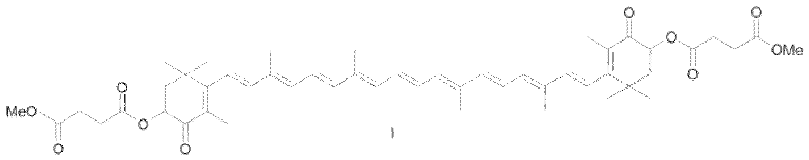 comprenant les étapes de procédé :
a) la mise en réaction d'un dialdéhyde en C₁₀ de formule II 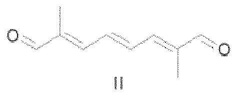 par une réaction de Wittig double avec un sel de phosphonium en C₁₅ de formule III 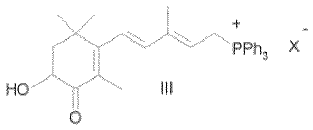 dans laquelle X⁻ est l'anion d'un acide organique ou inorganique HX,
dans un solvant comprenant du dichlorométhane, par ajout d'une solution d'un alcoolate de métal alcalin ou alcalino-terreux M¹OR¹ ou M² (OR¹)₂ dans un alcool R¹OH en tant que base,
M¹ représentant Li, Na, K ou Rb,
M² représentant Mg, Ca, Sr ou Ba,
et
R¹ représentant méthyle, éthyle, n-propyle ou isopropyle,
pour former une astaxanthine de formule IV 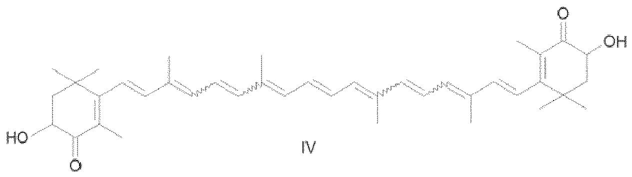 dans laquelle les deux doubles liaisons nouvellement formées se présentent aussi bien en configuration Z qu'en configuration E,
éventuellement suivie par un traitement aqueux du mélange réactionnel pour éliminer les sels M¹X ou M²X₂ ;
b) l'élimination de l'alcool R¹OH du mélange réactionnel de l'étape de procédé a) par lavage répété du mélange réactionnel avec de l'eau ou par élimination par distillation d'un mélange de solvants contenant du dichlorométhane et l'alcool R¹OH,
éventuellement suivie par le séchage du mélange réactionnel par élimination de l'eau par distillation azéotropique de dichlorométhane et d'eau ;
c) la mise en réaction de l'astaxanthine de formule IV, qui se trouve dans le mélange réactionnel traité à l'étape de procédé b), dans du dichlorométhane par ajout de chlorure de méthylsuccinoyle de formule V 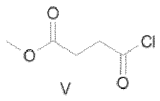 et d'une base azotée organique pour former un disuccinate diméthylique d'astaxanthine de formule Ia 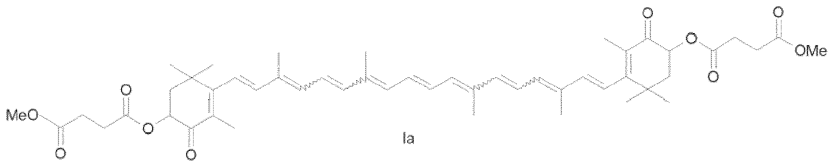
d) le traitement aqueux du mélange réactionnel de l'étape de procédé c) pour éliminer les sels solubles dans l'eau ;
e) le remplacement du solvant dichlorométhane par un alcool R²OH par élimination du dichlorométhane du mélange réactionnel de l'étape de procédé d) par distillation et ajout de l'alcool R²OH au mélange réactionnel, R² représentant méthyle, éthyle, n-propyle ou i-propyle ;
f) l'isomérisation thermique du disuccinate diméthylique d'astaxanthine de formule Ia fabriqué à l'étape de procédé c) pour former le disuccinate diméthylique d'astaxanthine all-E de formule I par chauffage du mélange réactionnel contenant R²OH de l'étape de procédé e) à au moins 50 °C, le mélange réactionnel présentant une teneur en eau de 10 à 90 % en volume d'eau par rapport au volume total du mélange réactionnel, et le disuccinate diméthylique d'astaxanthine all-E de formule I se formant sous forme cristalline et étant ensuite éventuellement isolé.

2. Procédé selon la revendication 1, dans lequel la base à l'étape de procédé a) est une solution de méthanolate de sodium dans du méthanol.

3. Procédé selon la revendication 2, dans lequel le méthanol est éliminé du mélange réactionnel à l'étape de procédé b) par lavage continu du distillat de dichlorométhane contenant du méthanol avec de l'eau dans un appareil mélangeur-décanteur.

4. Procédé selon l'une quelconque des revendications 1 à 3, dans lequel la base azotée organique à l'étape de procédé c) est la pyridine ou un dérivé de pyridine.

5. Procédé selon l'une quelconque des revendications 1 à 4, dans lequel l'alcool R²OH à l'étape de procédé e) est le méthanol.

6. Procédé selon la revendication 5, dans lequel l'isomérisation thermique à l'étape de procédé f) est réalisée dans du méthanol aqueux entre 80 et 120 °C sous la pression inhérente dans un appareil fermé.

7. Procédé selon l'une quelconque des revendications 1 à 6, dans lequel la teneur en eau défini à l'étape de procédé f) est ajustée par ajout d'eau au mélange réactionnel après l'étape de procédé e).
